# EUROPEAN PATENT APPLICATION

(11) **EP 4 180 804 A1**
(43) Date of publication of application: **17.05.2023**
(21) Application number: 21383028.4
(22) Date of filing: 12.11.2021
(51) Int. Cl.: G01N 27/327

(54) **ELECTROCHEMICAL SENSORS MADE OF PLASMA TREATED POLYLACTIC ACID (PLA)**

(71) Applicant: Universitat Politècnica De Catalunya, 08034 Barcelona (ES)
(72) Inventor: FONTANA ESCARTÍN, Adrian, 08034 BARCELONA (ES); LANZALACO, Sonia, 08034 BARCELONA (ES); BERTRAN CANOVAS, Oscar, 08034 BARCELONA (ES); ALEMÁN LLANSÓ, Carlos, 08034 BARCELONA (ES)
(74) Representative: Ungria López, Javier

(57) **Abstract**

A 3D-shaped electrode made of polylactic acid (PLA) for an electrochemical sensor, wherein the PLA is modified by a plasma treatment, and wherein the 3D-shaped electrode is a 3D-printed (e.g. FDM) electrode or a 3D-moulded electrode. Also the use of a 3D-shaped electrode as an electrochemical sensor is disclosed. Finally, a method for preparing a 3D-shaped electrode made of polylactic acid (PLA), the method comprising the steps of (a) 3D-shaping a PLA part, wherein the 3D-shape is obtained by 3D-printing or by 3D-moulding; and (b) treating the PLA part under plasma, is disclosed. Applications are the detection of dopamine and glucose wherein the enzyme glucose oxidase is immobilized on the polylactic acid electrode.

## Description

### FIELD OF THE INVENTION

This invention relates to the field of electrochemical sensors. More particularly, the invention relates to the field of electrochemical sensors made of polylactic acid (PLA) and which are manufactured by 3D-shaping technologies, such as 3D-printing and 3D-moulding.

### BACKGROUND OF THE INVENTION

Various diseases and health conditions require that patients closely monitor the concentration of molecules of biological interest in certain bodily fluids (e.g., blood, urine, etc.). Dopamine and glucose are examples of such molecules of biological interest, whose concentration may be altered in specific pathological contexts. In many cases, these types of molecules can be detected by means of electroanalytical methods, which require the use of specialised sensors.

Dopamine is a key catecholamine neurotransmitter with critical roles in the function of the human central nervous system. Abnormal levels have been related with several neurological diseases and disorders. For example, high levels of dopamine indicate hypertension and drug addictions, while low levels of dopamine are associated to depression, schizophrenia and Parkinson's disease. Therefore, there is a need to provide platforms for the detection of dopamine in a wide range of concentrations. Within this context, electrochemical dopamine sensors based on conducting nanomaterials (such as carbon nanotubes, graphene, metallic nanoparticles and conducting polymers) have been reported.

Glucose is a simple sugar with the molecular formula C₆H₁₂O₆. In energy metabolism, glucose is a key source of energy and circulates in the blood as blood sugar. The glucose blood content is regulated by the hormones insulin, incretin and glucagon, wherein insulin decreases glucose blood levels whereas glucagon increases them. Diabetes is a metabolic disorder wherein the body cannot properly regulate blood sugar levels either because of a lack of insulin or because of failure, by cells in the body, to respond to insulin. Patients with diabetes are often at risk of hyperglycaemia (high sugar blood levels) and/or hypoglycaemia (low sugar blood levels). For this reason, patients suffering from diabetes typically use glucose sensors in order to frequently check their sugar blood levels and manage the disease. Glucose sensors have several applications in the fields of clinical diagnosis, food industry and biotechnology even though nowadays they are mostly used for detection/management of diabetes and to prevent heart failure and cardiovascular diseases in non-diabetic patients. Glucose sensors are categorised as enzymatic and non-enzymatic. The majority of commercial sensors are enzymatic and based on glucose oxidase (GOx), which oxidizes glucose to gluconolactone. This is due to the high selectivity and sensitivity of enzymatic sensors, and also to the fact that non-enzymatic sensors rely on the use of very specific materials which require nanostructure optimisation in order to exhibit high electrocatalytic activity and effective electron transport from the electrocatalyst to the conductive electrode substrate.

Thus, electrochemical sensors are very useful to make quantitative assessment of various analytes, including biomolecules. These sensors are capable of detecting oxidation and reduction processes. This is because the presence of an analyte at the surface of conductive electrode materials (i.e. working electrode in electrochemical sensors) can cause changes in the potential and/or current, which is taken as a function of the analyte concentration. Among the methods used for electrochemical detection, potentiometry, cyclic voltammetry, differential pulse voltammetry, square wave voltammetry and chronoamperometry are widely used. In comparison to conventional techniques based on spectrometric and chromatographic methods, which provide high sensitivity but are time consuming and expensive, electrochemical detection methods show rapid response and are simple, sensitive and low-cost. Because of these advantages, the use of electrochemical sensors is commercially well-established at present time and several different methods exist for their fabrication.

Additive-manufacturing (3D-printing) is a fabrication procedure based on melting and solidification widely used for production of engineering components. Among 3D-printing technologies, fused deposition modelling (FDM) is the most common and inexpensive. FDM is based on fusing a continuous filament of a thermoplastic material fed through a heated printer extruder and depositing said thermoplastic material onto a substrate, where it is allowed to solidify in order to form layers. This results in the creation of the desired component. FDM benefits include having a large variety of materials to choose form, fast printing time, accurate dimension and good surface finishing. In recent years, FDM has been extended to the fabrication of electrochemical devices, including electrochemical sensors. The use of FDM technology in the fabrication of electrochemical sensors provides the following advantages: simple manufacturing using desktop-sized equipment, customised design in terms of size and geometry, fast prototyping and scalable manufacturing, high accuracy and uniformity, environment-friendly production without chemicals, solvents and waste, and transferability between 3D platforms from different manufacturers. However, the applicability of 3D-printing to the fabrication of electrochemical sensors is restricted by the availability of conductive thermoplastic filaments capable of carrying an electric flow, which are necessary to produce conducting working electrodes.

In order to overcome this limitation, conductive filaments are sometimes produced from composite materials obtained by mixing insulating thermoplastic materials (e.g. polylactic acid (PLA) and/or acrylonitrile butadiene styrene (ABS)) with carbon-based materials (e.g. graphene, carbon nanotubes and/or carbon black). However, the preparation and development of printable conductive filaments is not a simple task, since the fraction and distribution of the components must be tightly controlled to achieve printed electrodes with appropriate conductivity. Indeed, recent studies have shown that the conductivity of printed composite electrodes heavily relies on the printing parameters, which greatly affect the electrochemical performance of the sensor.

A promising alternative to the use of conductive composite filaments to manufacture electrochemical sensors is the implementation of post-processing strategies to 3D-printed non-conductive thermoplastic parts, wherein the post-processing treatment provides the required conductivity and electrochemical response. Recently, a flexible electrochemical biosensor was developed using a simple approach, which consisted on the surface activation of 3D printed non-conductive PLA specimens by immersion of the PLA parts in a NaOH solution and the subsequent surface polymerization of a conducting polymer (poly(3,4-ethylene dioxythiophene), also referred to as PEDOT) (please see Fontana-Escartin A, et al., Manufactured Flexible Electrodes for Dopamine Detection: Integration of Conducting Polymer in 3D-Printed Polylactic Acid, Advanced Engineering Materials 23(6), March 2021).

In view of the above, there is a need to provide alternative methods for the fabrication of convenient, inexpensive electrochemical sensors capable of accurately determining the concentration of molecules of biological interest in bodily fluids such as blood or urine.

### SUMMARY OF THE INVENTION

In this context, the inventors have found that electrochemical sensors can be effectively manufactured from polylactic acid (PLA) parts obtained by means of fused deposition modelling (FDM), wherein the PLA parts can be rendered conductive after being subjected to a low oxygen pressure plasma treatment (e.g., application of an electric power discharge of 100W for 2 minutes at a low pressure of O₂), without the need of incorporating any conducting additives to the sensors at any stage during the manufacturing process. Performance of the electrochemical sensors obtained by the proposed method has been successfully tested in the individual determination of dopamine, and in the simultaneous determination of dopamine and glucose by means of voltammetric assays.

Thus, in a first aspect, the invention relates to a 3D-shaped electrode made of polylactic acid (PLA), wherein the PLA is modified by a plasma treatment, and wherein the 3D-shaped electrode is a 3D-printed electrode or a 3D-moulded electrode. In a further aspect, the invention relates to the use of a 3D-shaped electrode according to the first aspect of the invention, as an electrochemical sensor. In a final aspect, the invention relates to a method for preparing a 3D-shaped electrode made of polylactic acid (PLA), the method comprising: (a) 3D-shaping a PLA part, wherein the 3D-shape is obtained by 3D-printing or by 3D-moulding; and (b) treating the PLA part under plasma.

### FIGURES

Figure 1: Procedure used to manufacture PLA(#)* sensors: (a) 3D-printing of PLA parts; (b) connection to GCEs; and (c) application of an electric discharge of 100 W during 2 min within a chamber with a low pressure of O₂ (# = 0.4, 0.6 or 0.8 mbar).
Figure 2: (a) FTIR spectra of PLA, PLA(0.4)*, PLA(0.6)* and PLA(0.8)* parts. (b) Raman spectra of PLA and PLA(0.8)*.
Figure 3: High resolution C 1s (left) and O 1s (right) spectra for untreated and treated PLA parts.
Figure 4: Representative SEM micrographs of (a) PLA with size bar of 1µm and 200nm; and (b) PLA(0.8)* with size bar of 1µm and 200nm. (c) Cyclic voltammograms recorded for PLA and PLA(0.8)* in PBS at a scan rate of 35 mV/s using a potential window from -0.90 to 0.90 V (left) and from -0.50 to 0.50 V (right).
Figure 5: High resolution C 1s (top), O 1s (middle) and N 1s (bottom) spectra for untreated and plasma treated GCE parts.
Figure 6: Representative low- and high-magnification SEM micrographs (left and right, respectively) of (a) GCE with size bar of 10µm and 300nm; and (b) GCE(0.8)* with size bar of 10µm and 300nm. (c) Cyclic voltammograms recorded for GCE and GCE(0.8)* in PBS at a scan rate of 35 mV/s using a potential window from -0.9 to 0.9 V (left) and from -0.5 to 0.5 V (right).
Figure 7: Electrochemical setup used for dopamine (DA) and/or for glucose (GL) detection.
Figure 8: Detection of DA in PBS using the PLA(0.8)* sensor by (a, b) cyclic voltammetry (CV), (c, d) linear sweep voltammetry (LSV) and (e, f) chronoamperometry (CA). (a) Cyclic voltammograms, (c) linear voltammograms and (e) chronoamperograms were recorded for different DA concentrations. Calibration plots, which represent current density versus DA concentration are shown for (b) CV, (d) SLV and (f) CA.
Figure 9: For PLA(0.8*) sensor: (a) cyclic voltammograms of DA in 0.1 M PBS (from 0 to 1000 µM) in 0.1 M PBS at pH 7.4 at a scan rate of 35 mV/s between -0.50 to 0.50 V; and (b) calibration curve of DA using the current density at the oxidation peak potential of the voltammograms displayed in (a).
Figure 10: (a, c) Detection of GL in PBS using the GOx-functionalised PLA(0.8)* sensor by CV and (b, d) the corresponding calibration plots. Graphics displayed in (a, b) correspond to the potential interval from -0.90 to 0.90 V, while those in (c, d) refer to the interval from -0.50 to 0.50 V.
Figure 11: (a) Simultaneous detection of DA and GL in PBS using the PLA(0.8)* sensor by CV using a potential interval from -0.90 to 0.90 V and (b) the corresponding calibration plots.
Figure 12: (a) Simultaneous detection of DA and GL in PBS using the PLA(0.8)* sensor by CV using a potential interval from -0.50 to 0.50 V and (b) the corresponding calibration plots.

### DETAILED DESCRIPTION

As explained above, the inventors have found that electrochemical sensors can be effectively manufactured from polylactic acid (PLA) parts obtained by means of fused deposition modelling (FDM), wherein the PLA parts can be rendered conductive after being subjected to a low oxygen pressure plasma treatment (e.g., application of an electric power discharge of 100W for 2 minutes in a chamber at a low pressure of O₂), without the need of incorporating any conducting additives to the sensors at any stage during the manufacturing process. Performance of the electrochemical sensors obtained by the proposed method has been successfully tested in the individual determination of dopamine, and in the simultaneous determination of dopamine and glucose by means of voltammetric assays.

### 3D-shaped electrode of the invention

In a first aspect, the invention relates to a 3D-shaped electrode made of polylactic acid (PLA), wherein the PLA is modified by a plasma treatment, and wherein the 3D-shaped electrode is a 3D-printed electrode or a 3D-moulded electrode.

In the context of the present invention, the term "electrode" refers to an electrical conductor that makes contact with the non-metallic parts of a circuit, such as an electrolyte. In a particular embodiment of the invention, the electrode of the invention is generally intended for use as an electrochemical sensor. Thus, in a particular embodiment, the electrode is an electrochemical sensor. As used herein, "electrochemical sensors" are a class of chemical sensors in which an electrode is used as a transducer element in the presence of an analyte, such that electrochemical sensors convert the information associated with electrochemical reactions (the reaction between an electrode and an analyte) into a qualitative or quantitative signal. It follows that the electrodes of the invention are suitable for use in electroanalytical methods. Electroanalytical methods are analytical chemistry techniques wherein analytes are studied by measuring the potential (volts) and/or current (amperes) in an electrochemical cell containing the analyte. There are different types of electroanalytical methods depending on which parameters of the cell are controlled (i.e., fixed) and which parameters are measured. The three main categories are potentiometry (the difference in electrode potentials is measured), coulometry (the cell's current is measured over time), and voltammetry (the cell's current is measured while actively altering the cell's potential).

In particular embodiments, the electrodes of the invention are used in amperometric/ voltammetric techniques. As used herein amperometry/ voltammetry are electrochemical techniques wherein information about an analyte is obtained by measuring the current as a function of the applied potential. In the context of the present invention, the methods of electrochemical detection can be, but are not limited to, cyclic voltammetry (CV), linear sweep voltammetry (LSV), chronoamperometry (CA), differential pulse voltammetry (DPV), and square wave voltammetry (SWV). Amperometric/ voltammetric techniques are conducted in a setup with at least two electrodes. Modern amperometric/ voltammetric techniques typically use a three-electrode system comprising a working electrode, a reference electrode and an auxiliary electrode. The working electrode is in contact with the analyte, applies the desired potential in a controlled way and facilitates the transfer of charge to and from the analyte. The electrode of the invention can be used as a working electrode in a three-electrode setup. The reference electrode is a half cell with a known reduction potential, which simply acts as reference in measuring and controlling the working electrode's potential, but does not pass any current. In the context of the invention, the reference electrode can be, but is not limited to, an Ag|AgCl (KCI, 3M) electrode. Other reference electrodes such as standard hydrogen electrode (SHE) and saturated calomel electrode (SCE) can also be used. The auxiliary electrode passes all the current needed to balance the current observed at the working electrode. In the context of the invention, the auxiliary electrode can be, but is not limited to, a platinum wire. Other auxiliary electrodes fabricated from an electrochemically inert material (e.g. gold, platinum or carbon) can be also used.

In the context of the invention, the term "polylactic acid", also known as poly(lactic acid) or polylactide (abbreviation PLA) refers to a thermoplastic polyester with backbone formula (C₃H₄O₂)ₙ or [-C(CH₃)HC(=O)O-]ₙ, and with CAS number 26100-51-6.

As used in the context of the present invention, the terms "3D-shaped" or "3D-shaping" generally refer to methods capable of giving a material a pre-determined, specific shape. 3D shaping can be achieved by means of fabrication methods such as 3D printing and 3D moulding. As used herein, the terms "3D printed" or "3D printing", which in the context of the present invention are used interchangeably with the term "additive manufacturing", refer to a variety of fabrication methods wherein computer-aided-design (CAD) software, 3D object scanners, or similar is used to instruct hardware to deposit material layer upon layer to create a three-dimensional object, in precise geometric shapes. 3D printing is the construction of a three-dimensional object from a CAD model or a digital 3D model. Methods of 3D printing include, but are not limited to, fused deposition modelling (FDM), photo-polymerisation and powder sintering. In a particular embodiment, the electrode is 3D-printed by fused deposition modelling (FDM). Fused deposition modelling (FDM), also known as fused filament fabrication (FFF) or filament freeform fabrication, is a 3D printing process that uses a continuous filament of a thermoplastic material. Filament is fed from a reel through a heated printer extruder head, and is deposited onto the growing piece. The print head is moved under computer control (i.e., computer-aided-design (CAD) software, 3D object scanners, or similar) to define the printed shape. Usually the head moves in two dimensions to deposit one horizontal plane, or layer, at a time. The work or the print head is then subsequently moved vertically by a small amount to begin a new layer. As used herein, the term "3D moulding" refers to fabrication methods wherein a liquid or freely flowing material is deposited into a cast. The cast is a negative form of the intended three-dimensional shape of the final object, such that, when the liquid or freely flowing material is allowed to solidify within the cast, the final object adopts the intended shape. In other words, thermoplastic moulding is a reversible moulding process by which pellets of plastic are melted, forced into a mould to assume their final shape and then quickly cooled to harden. Thermoset material is injected into a hot mould and then cooled to maintain the final shape of the part. The most popular techniques in plastic moulding are rotational moulding, injection moulding, blow moulding, compression moulding, conformation moulding, extrusion moulding, and thermoforming.

In the context of the invention, the term "plasma" refers to one of four fundamental states of matter (solid, liquid, gas and plasma). Plasmas can generally be generated by adding energy (e.g., electrical energy) to a gas or gas mixture. Upon exposure of a gas or gas mixture to an electrical energy source, the gas molecules are ionized, creating plasma which is highly electrically conductive and can interact with any surface. As used herein, the term "surface treatment" or "plasma treatment" refers to the procedure wherein the surface of an object is placed in contact with plasma such that the plasma is capable of modifying the surface of the object. A plasma treatment is usually performed in a chamber or enclosure that is evacuated under vacuum conditions so that air within the chamber or enclosure is pumped out prior to letting in any gas or gas mixture. The gas then flows into the chamber or enclosure at a pre-defined pressure. The process is carried out before any electrical energy is applied. At atmospheric pressure, plasma is typically very hot. However, plasma can be produced at low temperatures if pressure is reduced (for example, if pressure is reduced to e.g. under 1.0 mbar). These types of plasma are sometimes referred to as "cold plasma" or, more precisely, non-thermal plasma. Plasma treatments performed at low temperatures can easily process materials that are heat sensitive. In these cases, the material treated with low-pressure plasma is only slightly heated because the gas has a very low temperature.

The effects of plasma treatment on the surface of an object can be finely tuned by adjusting parameters such as the composition and/or pressure of the gas or gas mixture, the time of exposure of the surface to the plasma, and the voltage. Thus, in a particular embodiment, the gas or gas mixture in the plasma treatment is air or oxygen. The present invention can also be carried out in the presence of other gases, which allow the incorporation of oxygen bearing groups such as carbon dioxide and with non-oxygen containing gases such as argon and nitrogen. It is known that surface functionalisation in plasma treatment occurs through extremely reactive free radical mechanisms via peroxy and hydro-peroxy intermediates. In the case of non-oxygen containing gases the incorporation of oxygen is due to the presence of traces of oxygen in the chamber or due to post-treatment oxidation in air. In a particular embodiment, the plasma is atmospheric pressure plasma. In another particular embodiment, the plasma is low pressure plasma. In preferred embodiments, the low pressure plasma is plasma at a pressure of less than 1.0 mbar, less than 0.8 mbar, less than 0.6 mbar, or less than 0.4 mbar. In particular embodiments, the low pressure plasma is plasma at a pressure of 0.2 to 1.0 mbar, of 0.3 to 1.0 mbar, of 0.4 to 1.0 mbar, of 0.5 to 1.0 mbar, of 0.6 to 1.0 mbar, of 0.7 to 1.0 mbar or of 0.9 to 1.0 mbar. In a preferred embodiment, the low pressure plasma is plasma at a pressure of 0.4, 0.6 or 0.8 mbar. In a more particular embodiment, the plasma is a low oxygen pressure plasma. In still another particular embodiment, the plasma treatment is a plasma treatment with a low oxygen pressure plasma, wherein the low oxygen pressure is an oxygen pressure between 0.2 and 1.0 mbar, preferably wherein the oxygen pressure is of at least 0.4, of at least 0.6, or of at least 0.8 mbar, more preferably wherein the oxygen pressure is at least 0.8 mbar. In particular embodiments, the surface of the object (i.e., the surface of the PLA piece) is treated under plasma treatment for a duration of 1-300 seconds, of 10-240 seconds, of 50-180 seconds, or of 100-120 seconds. In more particular embodiments, the surface of the object (i.e., the PLA piece) is treated under plasma treatment for a duration of 1 second, 2 seconds, 3 seconds, 4 seconds, 5 seconds, 6 seconds, 7 seconds, 8 seconds, 9 seconds, 10 seconds, 12 seconds, 15 seconds, 20 seconds, 25 seconds, 30 seconds, 40 seconds, 50 seconds, 60 seconds, 70 seconds, 80 seconds, 90 seconds, 100 seconds, 110 seconds, 120 seconds or more than 120 seconds. In particular embodiments, the surface of the object (i.e., the surface of the PLA piece) is treated under plasma treatment for a duration of 15 minutes as an upper time limit. In particular embodiments the power discharge is of 50 to 500W, of 60 to 400W, of 70 to 300W, of 80 to 200W, or of 100 to 120W, preferably of 100 to 120W, more preferably of 100W.

In particular embodiments, the 3D-shaped electrode of the invention is in electrical contact with a secondary electrode for connecting to an electrochemical setup. This secondary electrode is also known as a connector. The secondary electrode is not in direct contact with the solution containing the analyte to be assayed. The secondary electrode is an electrode selected from the group consisting of glassy carbon electrodes (GCEs), electrodes based on carbon paste, electrodes prepared from graphite powder and mineral oil, electrodes based on stainless steel, nickel, silver, gold and/or platinum, and combinations thereof. In a particular embodiment, the 3D-shaped electrode is in electrical contact with glassy carbon electrodes (GCEs) for connecting to an electrochemical setup.

In other particular embodiments, the 3D-shaped electrode of the invention further comprises at least one functionalised region. The functionalised region can be functionalised with an enzyme or with a non-enzymatic catalyst. In particular embodiments, the functionalising group can be an enzyme selected from, but not limited to, glucose oxidase (GOx), laccase (Lac) and horseradish peroxidase (HRP). In a specific embodiment, the 3D-shaped electrode of the invention further comprises at least one functionalised region, wherein at least one of said at least one functionalised region is a region functionalised with a glucose oxidase enzyme (GOx).

All the terms and embodiments described elsewhere herein are equally applicable to these aspects of the invention.

### Use as an electrochemical sensor

In a further aspect, the invention relates to the use of a 3D-shaped electrode according to the first aspect of the invention, as an electrochemical sensor. In particular embodiments of the invention, the 3D- shaped electrode of the invention is used as an electrochemical sensor for the detection of a single analyte, or for the multiplexed detection of two or more analytes. In particular embodiments, the 3D- shaped electrode of the invention is used as an electrochemical sensor for the detection of at least 1, at least 2, at least 3, at least 4, at least 5, at least 6, at least 7, at least 8, at least 9, at least 10 or more analytes. In particular embodiments, the 3D- shaped electrode of the invention is used as an electrochemical sensor for the detection of 1, 2, 3, 4, 5, 6, 7, 8, 9, 10 or more analytes. The 3D-shaped electrodes of the invention can be used as an electrochemical sensor for the detection of analytes such as dopamine, glucose, lactate, serotonin, urea, uric acid, aspartic acid, insulin and other analytes. In a particular embodiment, the 3D-shaped electrode of the invention is used as an electrochemical sensor for the detection of dopamine. As used herein, dopamine (DA), also known as 3,4-dihydroxyphenethylamine, is a neurotransmitter of formula (1).

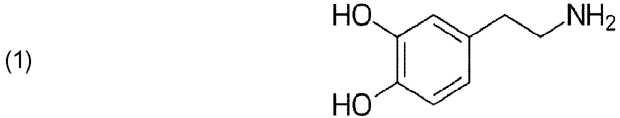

In another particular embodiment, the 3D-shaped electrode of the invention is used as an electrochemical sensor for the multiplexed detection of glucose and dopamine. As used herein, the term "glucose" refers to a simple sugar with the molecular formula C₆H₁₂O₆. Generally, the term "glucose" refers to the naturally occurring form of glucose, that is, D-glucose, but it also refers to L-glucose. In a particular embodiment of the invention, the term "glucose" refers to D-glucose.

All the terms and embodiments described elsewhere herein are equally applicable to these aspects of the invention.

### Preparation method

In a final aspect, the invention relates to a method for preparing a 3D-shaped electrode made of polylactic acid (PLA), the method comprising:
a) 3D-shaping a PLA part, wherein the 3D-shape is obtained by 3D-printing or by 3D-moulding;
b) treating the PLA part under plasma.

In a particular embodiment of the preparation method of the invention, the PLA part obtained in step (a) comprises at least one hole for putting the PLA part in electrical contact with at least one secondary electrode for connecting to an electrochemical setup. In a more particular embodiment of the preparation method of the invention, the PLA part obtained in step (a) comprises two holes for putting the PLA part in electrical contact with two secondary electrodes for connecting to an electrochemical setup. These secondary electrodes are not in direct contact with the solution containing the analyte to be assayed. The secondary electrodes are electrodes selected from the group consisting of glassy carbon electrodes (GCEs), electrodes based on carbon paste, electrodes prepared from graphite powder and mineral oil, electrodes based on stainless steel, nickel, silver, gold and/or platinum, and combinations thereof. In one particular embodiment of the preparation method of the invention, the PLA part coupled to at least one secondary electrode (e.g., GCEs) is treated under plasma in step (b).

In a particular embodiment of the preparation method of the invention, the PLA part obtained in step (a) further comprises at least one well for enhanced detection capacity. In a more particular embodiment of the preparation method of the invention, the PLA part obtained in step (a) further comprises at least two wells for enhanced detection capacity. The wells in the PLA part of the invention are well suited for functionalisation by coupling the surface of any or all of said at least one or at least two wells with specific enzymes or non-enzymatic catalytic groups such that the electrochemical sensor can detect the product resulting from the enzymatic reaction or the non-enzymatic catalytic reaction. Enzymes suitable for the functionalisation of the at least two wells can be, but are not limited to glucose oxidase (GOx), laccase (Lac) and horseradish peroxidase (HRP). Thus, in particular embodiments of the preparation method of the invention, the method comprises, after step (b), a further step (c) of coupling at least one of the wells with an enzyme. In a particular embodiment of the preparation method of the invention, the method comprises, after step (b), a further step (c) of coupling at least one of the wells with a Glucose Oxidase (GOx) enzyme.

In the preparation method of the invention, a PLA part is provided by 3D-shaping (i.e., 3D-printing or 3D-moulding). Methods of 3D printing include, but are not limited to, fused deposition modelling (FDM), photo-polymerisation and powder sintering. In a particular embodiment, step (a) of 3D-shaping in the preparation method of the invention is a step of 3D-printing, wherein the 3D-printing is 3D-printing by fused deposition modelling (FDM).

In the preparation method of the invention, the PLA part is subjected to plasma treatment. The effects of plasma treatment on the surface of an object can be finely tuned by adjusting parameters such as the composition and/or pressure of the gas or gas mixture, the time of exposure of the surface to the plasma, and the voltage. Thus, in a particular embodiment, the gas or gas mixture in the plasma treatment is air or oxygen. In another particular embodiment, the plasma is atmospheric pressure plasma, or low pressure plasma. In preferred embodiments, the low pressure plasma is plasma at a pressure of less than 1.0 mbar, less than 0.8 mbar, less than 0.6 mbar, or less than 0.4 mbar. In particular embodiments, the low pressure plasma is plasma at a pressure of 0.2 to 1.0 mbar, of 0.3 to 1.0 mbar, of 0.4 to 1.0 mbar, of 0.5 to 1.0 mbar, of 0.6 to 1.0 mbar, of 0.7 to 1.0 mbar, or of 0.8 to 1.0 mbar. In a preferred embodiment, the low pressure plasma is plasma at a pressure of 0.4, 0.6 or 0.8 mbar. In a more particular embodiment, the plasma is a low oxygen pressure plasma. In still another particular embodiment, the plasma treatment is a plasma treatment with a low oxygen pressure plasma, wherein the low oxygen pressure is an oxygen pressure under 1.0 mbar, preferably wherein the oxygen pressure is of at least 0.4, of at least 0.6, or of at least 0.8 mbar, more preferably wherein the oxygen pressure is at least 0.8 mbar. In particular embodiments, the surface of the object (i.e., the surface of the PLA part) is treated under plasma treatment for a duration of 1-300 seconds, of 10-240 seconds, of 50-180 seconds, or of 100-120 seconds. In more particular embodiments, the surface of the object (i.e., the PLA piece) is treated under plasma treatment for a duration of 1 second, 2 seconds, 3 seconds, 4 seconds, 5 seconds, 6 seconds, 7 seconds, 8 seconds, 9 seconds, 10 seconds, 12 seconds, 15 seconds, 20 seconds, 25 seconds, 30 seconds, 40 seconds, 50 seconds, 60 seconds, 70 seconds, 80 seconds, 90 seconds, 100 seconds, 110 seconds, 120 seconds or more than 120 seconds. In particular embodiments, the surface of the object (i.e., the surface of the PLA part) is treated under plasma treatment for a duration of 15 minutes as an upper time limit. In particular embodiments the power discharge is of 50 to 500W, of 60 to 400W, of 70 to 300W, of 80 to 200W, or of 100 to 120W, preferably of 100 to 120W, more preferably of 100W.

In a particular embodiment, step (b) of plasma treatment in the preparation method of the invention is plasma treatment with a low oxygen pressure plasma, wherein the low oxygen pressure is an oxygen pressure under 1.0 mbar, preferably wherein the oxygen pressure is of at least 0.4, of at least 0.6, or of at least 0.8 mbar, more preferably wherein the oxygen pressure is at least 0.8 mbar.

All the terms and embodiments described elsewhere herein are equally applicable to these aspects of the invention.

It should be noted that, as used in the specification and in the appended claims, the singular forms "a", "an", and "the" include their plural referents unless the context clearly indicates otherwise. Similarly, the term "comprises" or "comprising" as used herein also describes "consists of" or "consisting of" in accordance with generally accepted patent practice.

### EXAMPLES

The instant invention is hereby described by way of the following examples, which are to be construed as merely illustrative and not limitative of the scope of the invention.

### Example 1: Materials and Methods

### Additive manufacture

PLA filaments were shaped using a BCN3D SIGMAX R19 3D printer equipment. Nozzles with 0.3 mm of diameter were used in all cases, whereas the printing temperature was set at 230 °C and the infill density at 100%. The device was designed using Cura software and the filaments were shaped as rectangular parallelepipeds of size 2.00 x 1.00 x 0.35 cm³. In order to increase the capacity for electrochemical detection, two rectangular wells of 2.0 x 2.0 x 0.5 mm³ were printed on all samples. Also, two holes of 1.5 x 1.5 x 5.0 mm³ were printed to facilitate the coupling of two GCEs, which are necessary for the sensing signal reach the electrochemical setup.

### Electric discharge treatment

PLA prototypes with (for electrochemical detection) or without (for characterisation) GCEs were put within a chamber with a volume of 1.7 dm³ and the whole system was purged under vacuum and filled with oxygen gas. More in detail, the system was evacuated until the desired pumping down pressure was reached, which corresponds to the final pressure of the purging step preceding the application of the electric discharge. Subsequently, the PLA surfaces were subjected to an electric discharge employing a generator with a maximum radio-frequency of 13.56 MHz and a maximum power output of 500 W. The gas pressure was changed from 0.4 to 0.6 and 0.8 mbar, with minimum flux of the gas of ∼ 20 sccm, automatically regulated by a valve to ensure the desired final pressure. All the experiments were carried out with a 100 W power supply or 2 min. After the treatment, all samples were stored under vacuum for a few days, if not used immediately.

### Characterisation

FTIR transmittance spectra were recorded on a FTIR Jasco 4100 spectrophotometer. Pellets were deposited on an attenuated total reflection accessory (Top-plate) with a diamond crystal (Specac model MKII Golden Gate Heated Single Reflection Diamond ATR). For each sample 64 scans were performed between 4000 and 600 cm⁻¹ with a resolution of 4 cm⁻¹. Raman spectra were obtained by the inVia Qontor confocal Raman microscope (Renishaw), equipped with a Renishaw Centrus 2957T2 detector and a 785 nm laser.

X-ray photoelectron spectroscopy (XPS) analyses were performed on a SPECS system. The spectrometer was equipped with a high-intensity twin-anode X-ray source XR50 of Mg/AI (1253 eV/ 1487 eV) operating with the Al anode at 150 W, placed perpendicular to the analyser axis, and using a Phoibos 150 MCD-9 XP detector. The position of the stage was digitally controlled to ensure that the spot was in the same exact place during the whole treatment. The pass energy of the hemispherical analyser was set at 25 eV and the energy step of high resolution spectra was set at 0.1 eV. The pressure in the analysis chamber was always below 10⁻⁷ Pa, and binding energy (BE) values were referred to the C 1s peak at 284.8 eV. Data were processed with the CasaXPS software (Casa Software Ltd., UK).

The water contact angle was determined using the sessile water drop method at room temperature and controlled humidity in order to establish the hydrophilicity of the surface. Measurements were carried with the equipment OCA 20 (DataPhysics Instruments GmbH, Filderstadt), and the software SCA20 was used to analyze the data acquired. Averages correspond to at least 10 measures for each studied system.

SEM studies were performed in a Focussed Ion Beam Zeiss Neon 40 scanning electron microscope equipped with an EDX spectroscopy system and operating at a voltage of 2 kV.

Electrochemical characterisation was performed using an Autolab potentiostat controlled by the NOVA software. A conventional three-electrode cell was used with the printed sensor, after electric discharge treatment, as working electrode, Ag|AgCl (KCI, 3M) reference electrode and platinum wire as counter electrode. The electrolyte employed was a phosphate buffer saline (PBS) solution (pH 7.4). Experiments were performed at 37 °C, using different initial, final and reversal potentials.

### Glucose oxidase (GOx) functionalisation

GOx was dialysed using a sodium acetate buffer solution (0.2 M at pH 3.6, which was prepared using sodium acetate and glacial acetic acid. GOx was dissolved (5 mg/mL) in sodium acetate buffer solution under soft agitation. Finally, the enzyme-containing solution was filtered by syringe filtration through a 0.45 µm membrane and dialysed into 6-8 kDa semipermeable membrane against the buffer overnight at 4 °C.

The immobilisation of the enzyme on chemically modified PLA was performed using a two-step process. Firstly, the substrate was activated by dropping into one of the wells of the PLA manufactured prototype a 4 mg/mL solution of 1-[3-(dimethylamino)propyl]-3-ethylcarbodiimide methiodide in sodium acetate buffer solution 0.1 M at pH 4.8 (5 µL). After 1 h at 4 °C, the activated electrode was washed three times in milli-Q water. In the second step, 4 µL of GOx solution (2, 5 or 10 mg/mL) in sodium acetate buffer solution 0.1 M pH 3.6 were drop casted into the well of the printed PLA sample. Then, the GOx-containing electrodes were dried overnight at 4 °C and, subsequently, washed three times using the same buffer.

### Electrochemical detection

The electrochemical detection of dopamine (DA) and glucose (GL) was studied by cyclic voltammetry (CV), linear sweep voltammetry (LSV) and chronoamperometry (CA) using the same three-electrode cell. For CV and LSV the scan rate was 5 and 35 mV/s, respectively, while the potential interval was explicitly specified in the text for each assay. Measurements were performed by adding different concentrations of DA and/or GL to a phosphate buffer saline (PBS) solution at pH 7.4. The potential for CA measurements was 0.50 V.

### Example 2: Preparation of the sensor

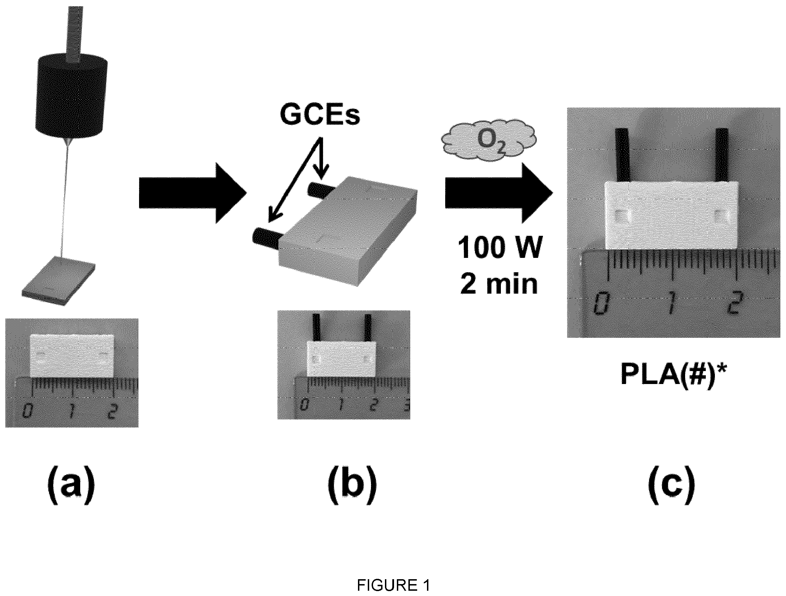

Figure 1 displays the procedure used in this work. First, PLA filaments were shaped at 230 °C using nozzles of 0.3 mm in diameter. The size of the printed prototypes was 2.0 x 1.0 x 0.35 cm³, two wells of 2.0 x 2.0 x 0.5 mm³ being embedded in all them to enhance the electrochemical detection capacity (Figure 1a). Also, two holes of 1.5 x 1.5 x 5.0 mm³ were printed to facilitate the coupling of two GCEs, which are necessary for the sensing signal reach the electrochemical setup (Figure 1b). Finally, PLA prototypes with (for electrochemical detection) or without GCEs (for characterisation) were introduced in a vacuum chamber and O₂ was blown until the pressure reached 0.4, 0.6 or 0.8 mbar. After applying an electric discharge with a power supply of 100 W during 2 min, the chemically transformed prototype was retired and used (Figure 1c). The chemically modified prototype, which acted as working electrode in the sensing process, was denoted PLA(#)*, where # indicates the pressure of O₂ used for the transformation of electrochemically inert PLA into an electrochemically active material. Comparison of the photographs recorded for PLA and PLA(0.8)* (Figure 1b-c) prove that the aspect and texture of the parts remain unaltered after treatment.

### Example 3: Characterisation of the sensor after electric discharge (i.e., plasma treatment)

The effect of the electric discharge treatment (i.e., plasma treatment) on the structure of PLA and GCEs have been studied separately for a better understanding of the chemical changes produced on the two components of the sensor.

### Characterisation of chemically transformed PLA

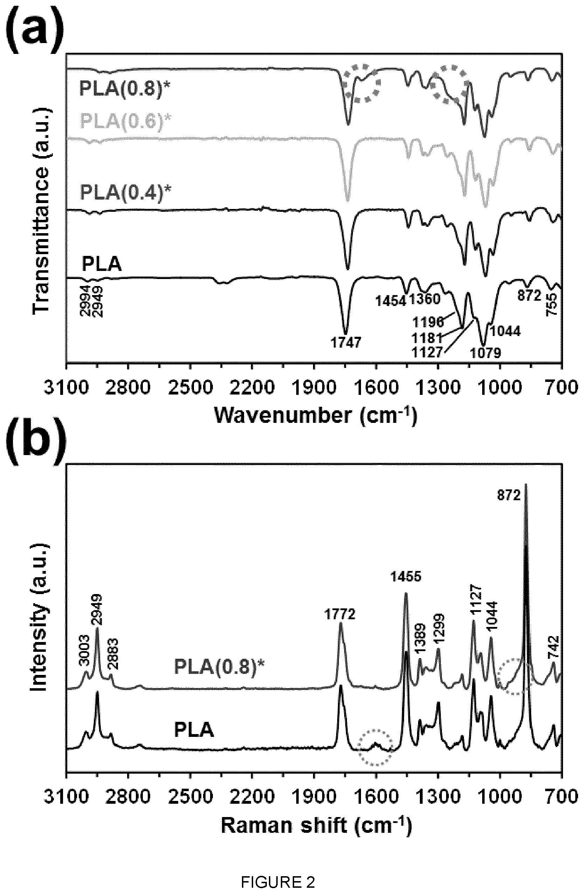

FTIR spectroscopy. Figure 2a compares the FTIR spectra of PLA, PLA(0.4)*, PLA(0.6)* and PLA(0.8)*. The PLA spectrum shows the more intense peaks at 1079 and 1127 cm⁻¹ for C-O stretching, 1360 cm-1 for -CH₃ symmetric bending, 1454 cm⁻¹ for -CH₃ asymmetric bending, and 1747 for C=O stretching. Other characteristic peaks appear at 1181 and 1196 cm⁻¹ for asymmetrical and symmetrical -C-O-C vibrations, 2949 cm⁻¹ for the C-H3 stretching and 2994 cm-1 for the C-H stretching. Finally, the bands at 872 and 755 cm⁻¹ are attributed to the C-C stretching of amorphous and crystalline phases, respectively, which explains the two components associated to the C-O stretching. Comparison with PLA(0.4)*, PLA(0.6)* and PLA(0.8)* spectra indicates that, in general, the transmittance of the vibrations related with the ester group increases with the pressure of oxygen applied during the electric discharge treatment. However, changes are particularly noticeable for the PLA(0.8)* spectrum, which reflect the formation of new functional groups. Thus, the peaks detected at 1670 and 1232 cm⁻¹ (dotted circles in Figure 2a), which are nor present in the spectra of PLA(0.4)* and PLA(0.6)*, are attributed to the C=O and C-O stretching modes of functional groups different from the ester group of PLA. This increment in the quantity of oxygenated species is consistent with the surface oxidation of PLA.

Raman spectroscopy. Similar conclusions were derived from Raman spectroscopy. This is illustrated in Figure 2b, which compares the Raman spectra of PLA and PLA(0.8)*. PLA shows peaks at 742 cm⁻¹ assigned to O-C=O in plane bending, 872 cm⁻¹ to C-COO vibration, 1044 cm⁻¹ to C-CH₃ stretching, 1127 cm⁻¹ to CH₃ asymmetric groups, 1299 cm⁻¹ to CH deformation, 1369 and 1455 cm⁻¹ to symmetric and asymmetric CH₃ deformation mode, respectively, 1742 cm⁻¹ to strong C=O groups, and 2883-3004 cm⁻¹ to C-H stretching modes of CH₃. Both the deformation of the signal at the region close to the 872 cm⁻¹ and the apparition of a broad band at 1600 cm⁻¹ in the PLA(0.8)* spectrum are consistent with the chemical transformation of PLA, which has been mainly attributed to the surface oxidation.

X-ray photoelectron spectroscopy (XPS). PLA, PLA(0.4)*, PLA(0,6)* and PLA(0.8)* were analyzed by XPS. The recorded survey spectra for such materials (not shown) have been analysed, while the atomic compositions are summarized in Table 1. The survey spectra indicated the presence of carbon (C 1s peak at 285 eV) and oxygen (O 1s peak at 533 eV). The presence of a small amount of nitrogen (N 1s peak at 400 eV), which ranges from 0.3% in PLA to 2.9% in PLA(0.4)* (Table 1), has been attributed to the additives of PLA filaments or adsorbed N₂ molecules in all cases with exception of PLA(0.4)*, for which a certain amount of nitrogen arose through contamination of the material during manipulation. The C/O ratio, which decreases from 3.2 in PLA to 2.1-2.5 in treated samples, reflects a chemical transformation that is consistent with a reduction in the amount of carbon, whereas the atomic composition of oxygen increases.

**Table 1. Atomic percent composition (C, O and N; in %) of PLA, PLA(0.4)*, PLA(0.6)*, and PLA(0.8)* as determined by XPS. The C/O and C/N atomic ratios are also indicated.**

| **Sample** | **C** | **O** | **N** | **C/O** | **C/N** |
|---|---|---|---|---|---|
| PLA | 75.8 | 23.9 | 0.3 | 3.2 | 252.7 |
| PLA(0.4)* | 69.1 | 28.0 | 2.9 | 2.5 | 27.6 |
| PLA(0.6)* | 67.4 | 32.3 | 0.3 | 2.1 | 224.7 |
| PLA(0.8)* | 70.0 | 29.3 | 0.7 | 2.4 | 100.0 |

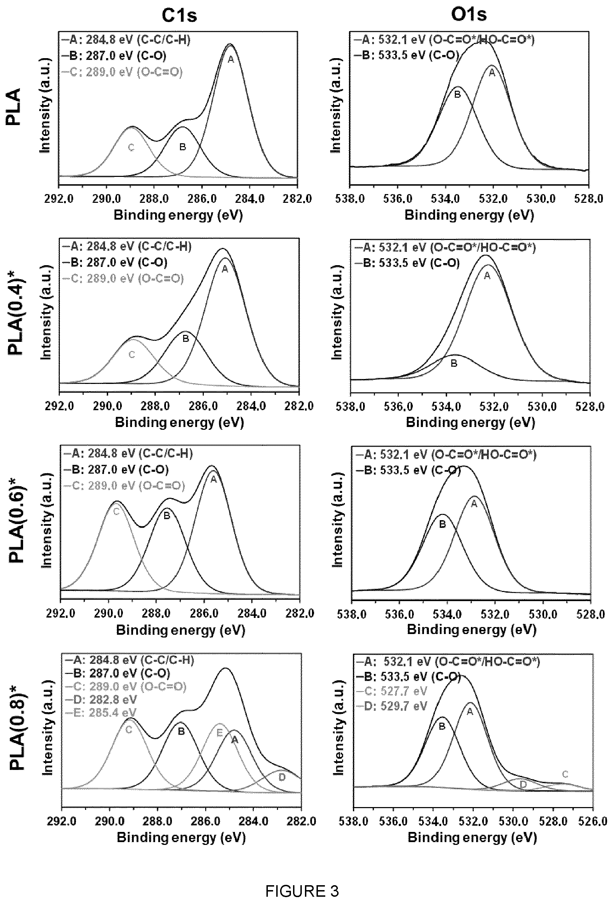

Figure 3 displays the deconvolution of the C 1s and O 1s peaks in the high resolution spectra recorded for the studied samples. The deconvolution of the C 1s peak led to three Gaussian curves, which were attributed to the existence of C-C/C-H (284.8 eV), C-O (287.0 eV) and O-C=O (289.0 eV) bonds. For untreated PLA, the contribution of such bonds, expressed as %, was significantly higher for C-C/C-H (57.2%) than for CO (21.7%) and O-C=O (21.1%). However, electric discharge treatment at different O₂ pressures changes such distribution by reducing the contribution of the C-C/C-H bonds and increasing the contribution of the C-O and O-C=O bonds. For PLA(0.8)*, two new Gaussian curves appear at 282.8 and 285.4 eV (Figure 3), exhibiting a contribution of 21.2% and 8.6%, respectively. These have been attributed to an etching effect occurring at the highest O₂ pressure, which promotes the formation of C-M bonds with the metallic atoms of the holder used to support the samples. The deconvolution of the high resolution O 1s peak into components is fully consistent with such observation, as is shown in Figure 3. Two components, which are attributed to the O=C-O (532.1 eV) and C-O (533.5 eV) bonds, are detected in all samples with exception of PLA(0.8)*. In this latter case, two additional curves, which are associated with the formation of metallic oxides induced by the etching effect produced at the highest pressure, are identified at 527.5 and 529.7 eV. Inspection to the distribution of the components reveals that the electric discharge treatment tends to reduce the contribution of the C-O curve whereas that of the O=C-O tends to increases. Overall, XPS observations are consistent with the chemical transformation induced by the applied treatment. On the other hand, the high resolution N 1s peak shows a single curve centred at 400.0 eV, independently of the treatment. Considering that the greatest chemical changes were obtained at the highest oxygen pressure, PLA(0.8)* samples were selected not only for further characterisation studies but also for the detection analyses.

Water contact angle. The surface hydrophilicity of PLA increased with the discharge treatment. Thus, the water contact angle decreased from 73° ± 1° for PLA to 48° ± 2° for PLA(0.8)*, which is fully consistent with the generation of polar oxygen-containing groups.

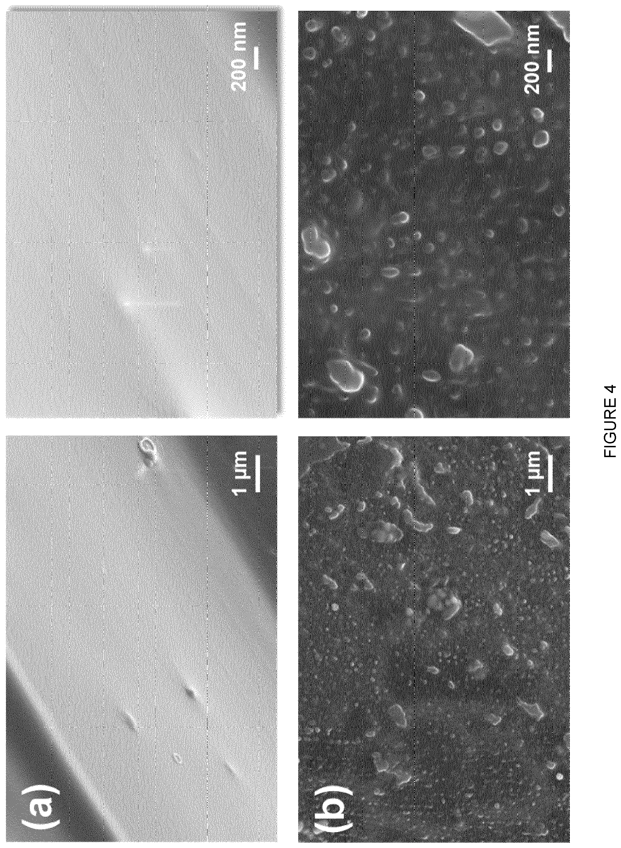

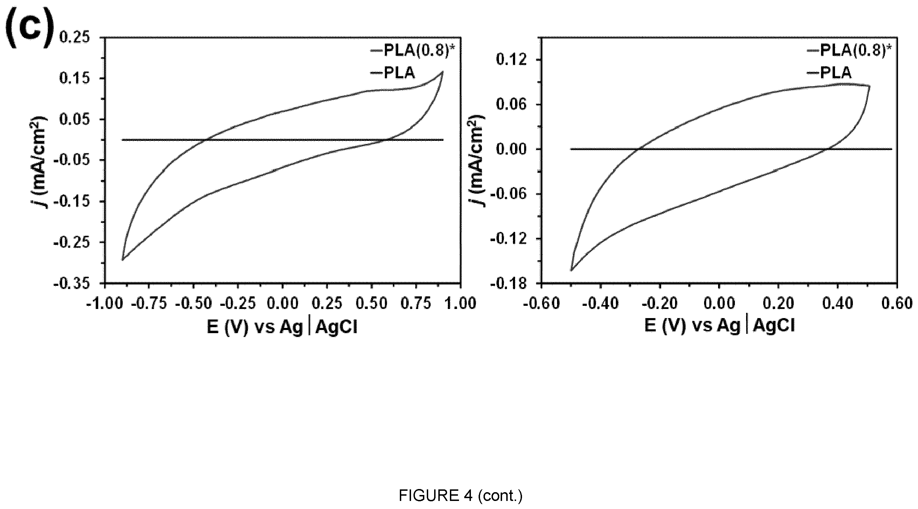

Scanning electron microscopy (SEM). Low and high-magnification SEM micrographs of PLA and PLA(0.8)* parts, which are compared in Figure 4, indicate that the chemical transformation produced by the discharge treatment in presence of oxygen is accompanied by a drastic change in the surface morphology. The homogeneous and smooth surface of PLA (Figure 4a) transforms into a heterogeneous rough surface after treatment (Figure 4b). This effect is expected to favour the electrochemical detection process, which is known to improve with increasing active surface area.

Electrochemical response. Figure 4c compares the electrochemical response of PLA and PLA(0.8)* parts, which was evaluated by cyclic voltammetry using a phosphate buffer saline (PBS) solution (pH 7.4). Two potential windows latterly used for the detection studies were scanned at a rate of 3.5 mV/s: from -0.90 to 0.90 V and from - 0.50 V to 0.50 V. As it can be seen, the electrochemical activity of PLA is zero, independently of the potential window. This behaviour is consistent with its compact homogeneous structure and its insulating nature, precluding ions from the PBS solution and/or electrons from redox processes on the PLA to reach the connected GCE. On the contrary, PLA(0.8)* exhibits significant electrochemical response for the two studied potential windows, as is evidenced by the area of the recorded voltammograms. The voltammetric charge accumulated over the shortest and largest potentials windows (0.16 and 0.42 mC, respectively) is comparable to that of conducting polymers, which are known to exhibit intrinsic electrochemical and electrical properties. Moreover, voltammograms obtained for PLA(0.8)* are symmetric indicating that oxidation and reduction processes are reversible.

### Characterisation of GCE

X-ray photoelectron spectroscopy (XPS). The recorded survey spectra (not shown) have been analysed, while the atomic compositions for GCEs before and after treatment in chamber with an O₂ pressure of 0.8 mbar, GCE(0.8)*, are shown in Table 2.

**Table 2. Atomic percent composition (C, O and N; in %) of GCE and GCE(0.8)* as determined by XPS. The C/O and C/N atomic ratios are also indicated.**

| **Sample** | **C** | **O** | **N** | **C/O** | **C/N** |
|---|---|---|---|---|---|
| GCE | 73.8 | 25.0 | 1.2 | 2.9 | 61.5 |
| GCE(0.8)* | 63.9 | 34.7 | 1.4 | 1.8 | 45.6 |

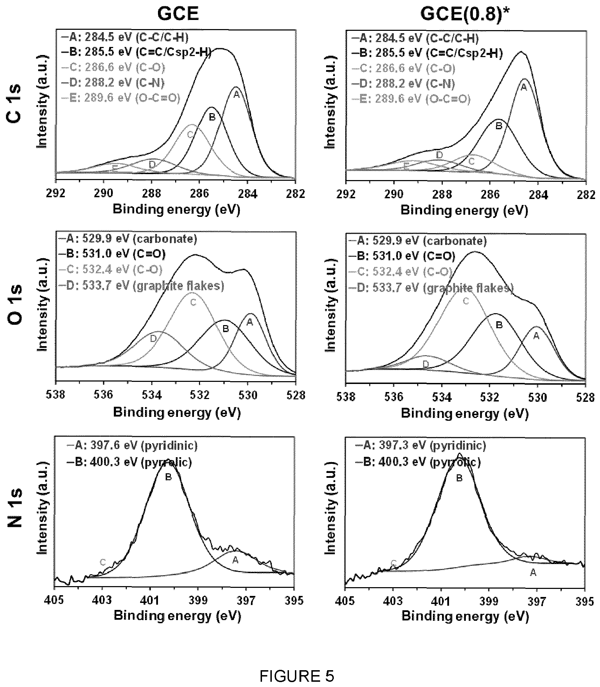

The spectrum recorded over the untreated GCE shows the presence of C and O and a minor contribution of N. This is in agreement with the compositions of GCE, which includes phenol, carbonyl, carboxyl, lactone and quinone functional groups. The electric discharge treatment results in a reduction of the C/O ratio, which is due to the activation in the O₂ atmosphere. Figure 5 displays the high resolution C 1s, O 1s and N 1s spectra for both GCE and GCE(0.8)* specimens. The curves at 284.5 and 285.5 eV have been attributed to Csp2 and Csp3 species, respectively, whereas those at higher binding energies are associated to C-O and C-N species. Similarly, the bands centred at 531.0 and 532.4 eV in the O 1s spectrum are ascribed to C=O and C-O bonds, respectively, while the ones at 529.9 and 533.7 eV have been associated to negatively charged molecules coming from atmospheric oxidation (CO₃²⁻) and positively charged particles (e.g. graphite flakes), respectively. Regarding to the latter, commercial GCE, which are formed by the pyrolysis of polymeric precursors, contains imperfect nanoparticles, such as graphite crystals and networks of stacked graphite sheets. The N 1s bands at 397.6 and 400.3 are ascribed to pyridinic and pyrrolic nitrogen atoms, respectively. Comparison among the spectra recorded for GCE and GCE(0.8)* reveals that the changes induced by the treatment are much less significant than for PLA. Thus, the main differences between GCE(0.8)* and GCE were ascribed to the incorporation of oxygen to Csp2 (O-C=O) and Csp3 (C-O) species.

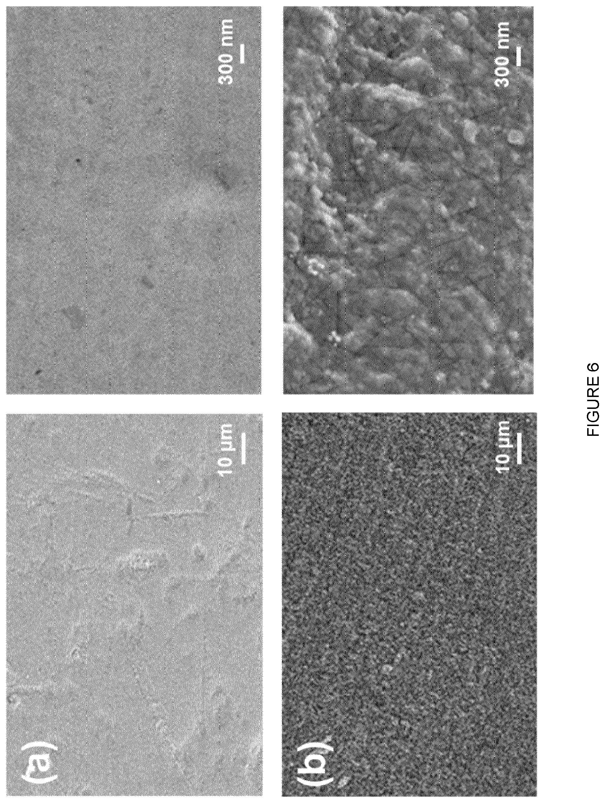

Scanning electron microscopy (SEM). SEM micrographs of bare GCE and GCE(0.8)*, which are compared in Figure 6a-b, indicate that, although the electric discharge treatment affects the texture of the surface, the change is much less drastic than for PLA. Thus, the only difference between the untreated and the treated samples is the appearance of some superficial scales in the latter, which are significantly less pronounced than the micrometric protuberances observed for PLA(0.8)*.

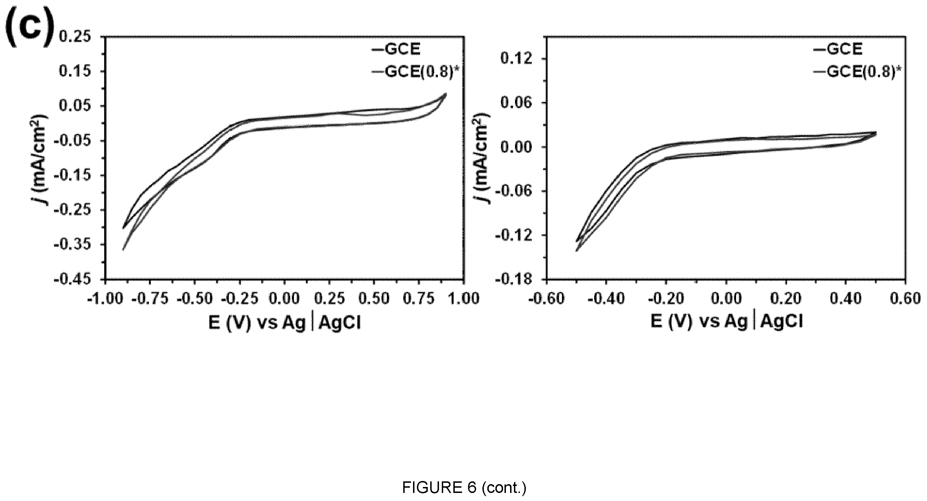

Electrochemical response. The small effect of the electric discharge treatment in GCE is also demonstrated in Figure 6c, which displays the cyclic voltammograms recorded for CGE and CGE(0.8)* using different potential intervals. Voltammograms are similar for the two electrodes, independently of the potential interval, indicating that electric discharges in presence of a low-pressure O₂ atmosphere do not alter the intrinsic conductivity and electrochemical behavior of GCE. Instead, the electrochemically inert PLA was completely transformed by such treatment (Figure 4c), transforming it into an electrochemically active plastic material. These features allowed us to apply the electric discharge treatment on the whole sensor (i.e., once the GCEs were coupled to the PLA parts), facilitating the manufacturing process.

### Example 4: Detection of dopamine (DA) and glucose (GL)

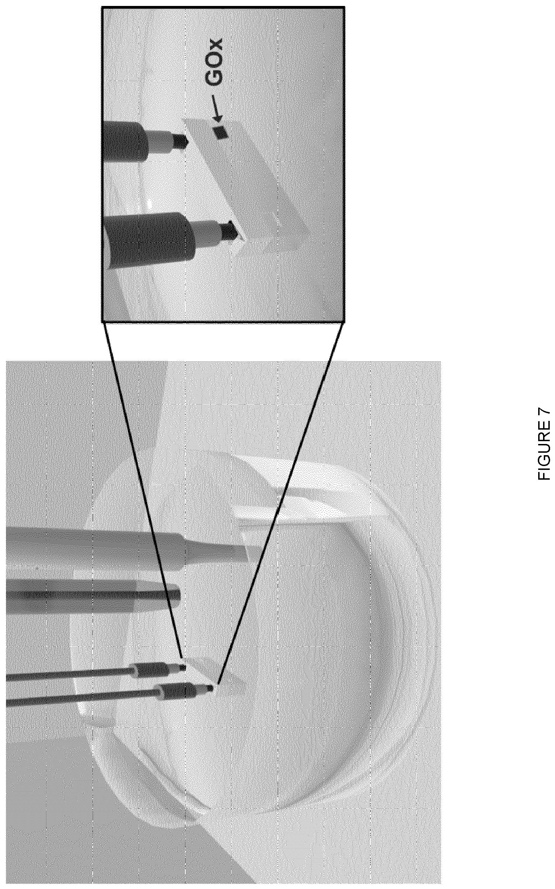

The electrochemical setup used for DA and/or GL detection consisted in a three-electrode cell in which the PLA(0.8)* sensor (Figure 1c), platinum wire and Ag|AgCl (KCI, 3M) were used as working, counter and reference electrode, respectively. Another important characteristic of the setup is that the two wells printed in sensor were immersed in the analyte(s)-containing electrolytic solution, whereas the GCEs used as connectors were not in contact with such solution (Figure 7). One of the two wells was functionalised with glucose oxidase (GOx) to catalyze the oxidation of GL to gluconolactone (see below) and while the other remained naked to facilitate the detection of DA. It should be mentioned that the signals recorded using sensors with two printed wells were much more sensitive than sensors prepared with only one well. This has been attributed to ability of modified PLA to detect DA in the two wells, thus increasing the signals of this analyte, meanwhile GL is only detected by the well functionalised with the enzyme. Results for the individual and simultaneous detection of DA and GL are discussed below.

### Detection of DA

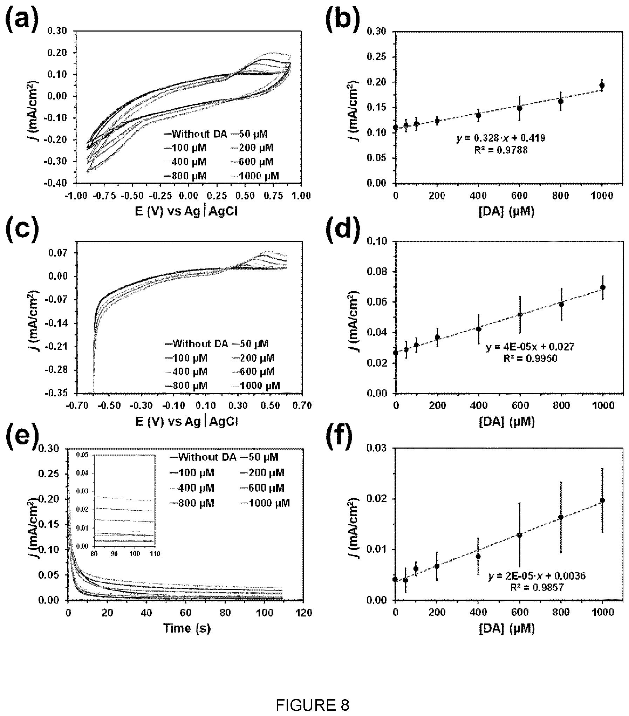

As a first step, the detection of dopamine DA alone was investigated using different electrochemical techniques. The oxidation of DA at PLA(0.8)* sensor was assessed by cyclic voltammetry (CV) in PBS (pH 7.4) containing different concentrations of DA at a scan rate of 35 mV/s between -0.90 to 0.90 V (Figure 8a). An oxidation peak, which is particularly pronounced for DA concentrations ≥ 100 µM, appears at 0.5 V, indicating electron and mass transfer processes become enhanced with increasing DA concentration. Thus, such current response corresponds to the oxidation of DA to dopaminoquinone (DQ) catalyzed by the chemically modified PLA, PLA(0.8)*. The calibration curve was obtained using the average current density at the oxidation peak potential of three independent measurements at each DA concentration (Figure 8b). Within the range of 50 µM to 1000 µM, the accuracy of the PLA(0.8)* sensor is very high, as is reflected by the linearity of the curve (i.e. R²= 0.979). On the other hand, the sensitivity that was derived from the slope of the calibration curve, is 0.328 mA/(cm²•µM), while the limit of detection (LOD), calculated as the ratio between three times the standard deviation of the blank (i.e. without DA) and the slope of the calibration curve, is 0.08 µM.

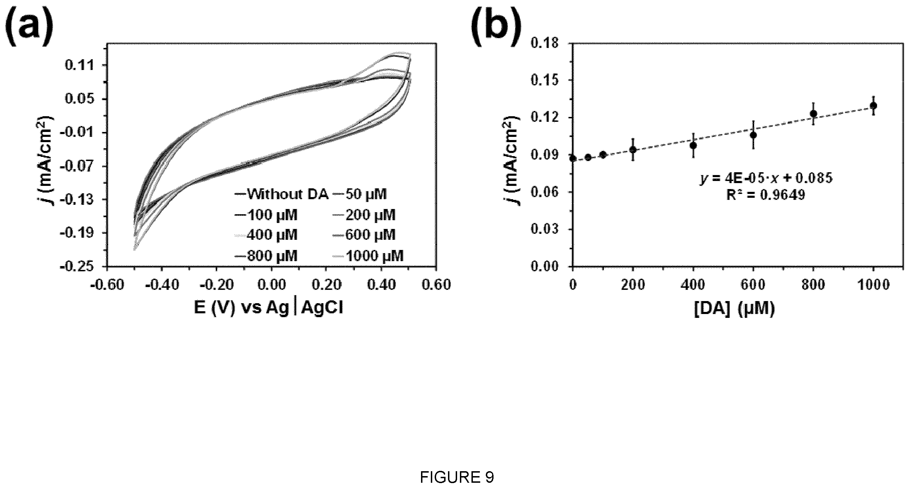

In order to explore the effect of the voltage interval in the detection of DA by CV, different studies were performed by reducing the initial and final potentials, as well as the reversal potential. An illustrative example is displayed in Figure 9, which shows the results obtained between -0.50 to 0.50 V. Although the sensitivity improved to 0.04 µA/(cm²•µM), the linearity of the calibration curve (R²= 0.965) and the LOD (31.1 µM) were worse than those obtained for the widest potential interval. A similar behaviour was obtained for the other explored potential intervals.

Simple linear sweep voltammetry (LSV), which measures the current density scanning the voltage from a lower limit to an upper limit, could be an alternative for the electrochemical detection of DA using the PLA(0.8)* sensor. Figure 8c displays the linear sweep voltammograms obtained for different DA concentrations in PBS (pH 7.4) by scanning the potential between -0.60 and 0.60 V at 5 mV/s. The DA oxidation peak is clearly detected at 0.4 V, the peak current density increasing linearly with the DA concentration (i.e. R²= 0.995), as is shown in the resulting calibration curve (Figure 8d). However, the LOD (280 µM) is much higher than that obtained by CV, indicating that, despite its simplicity, LSV is not the most appropriated method to exploit the full potential of the PLA(0.8)* sensor.

Finally, determination of DA was also evaluated using chronoamperometry (CA). Figure 8e displays the chronoamperograms at the fixed potential +0.50 V (vs. Ag/AgCI) with increasing concentration of DA. In all cases, after 60 s the current density stabilized at a constant value, which increases with the DA concentration. Figure 8f shows that the steady current density versus the DA concentration calibration plot was linear (R² = 0.9857). In this case, the LOD was 352 µM, evidencing that the efficacy of chronoamperometric techniques is significant worse than that of voltammetric ones, especially than CV.

### Detection of glucose (GL)

The GOx enzyme was coupled to the surface of one of the wells of the PLA(0.8)* sensor (Figure 1) by the carbodiimide method, which promoted the conjugation of the carboxyl groups generated at the surface of PLA after the discharge treatment to the amine groups of enzyme. For this purpose, the sensor was activated by immersing it into 4 mg/mL of 1-[3-(dimethylamino)propyl]-3-ethylcarbodiimide methiodide in 0.1 M sodium acetate buffer solution (pH 4.8) for 1 h at 4 °C under slight agitation. After that, the activated sensor was washed with milli-Q water and 4 µL of a GOx solution in 0.1 M sodium acetate buffer solution (pH 3.6) were deposited into the selected well. Although different GOx concentrations (2, 5 and 10 mg/mL) were considered for the solutions used in the step, results discussed below has been focused on sensors functionalised with the 10 mg/mL GOx solution, which provided the highest sensitivity and the lowest LOD. Finally, the GOx-containing sensors were dried overnight at 4 °C and washed three times using the same buffer solution. The success of this procedure for the covalent immobilisation of peptides and proteins, including GOx, has been extensively reported. In this work, the immobilisation of the enzyme was confirmed by XPS and FTIR spectroscopy, as shown in previous studies.

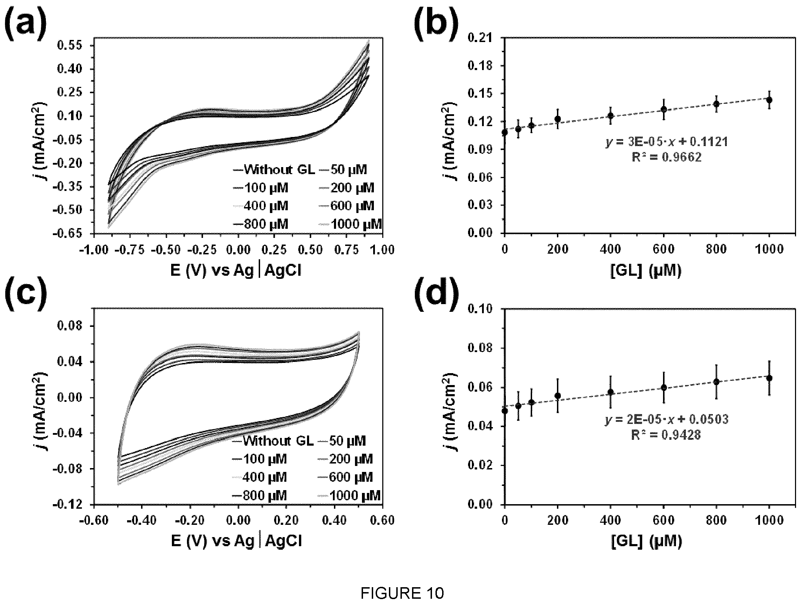

Figure 10a shows the cyclic voltammograms recorded for GOx-functionalised PLA(0.8)* sensors (10 mg/mL GOx) in PBS solutions with different GL concentrations using a potential interval from -0.90 to 0.90 V at a scan rate of 35 mV/s. The oxidation of GL is detected by the oxidation peak at -0.2 V, the peak current density increasing with the concentration of GL. This response corresponds to the catalytic activity of the PLA(0.8)* towards hydrogen peroxide (H₂O₂) generated by the enzymatic reaction. Thus, GOx specifically catalyzes the oxidation of glucose as follows:

(1) β-D-glucose + O₂ → D-gluconic acid + H₂O₂

and the chemically modified PLA by electric discharge treatment catalyzes the reaction of H₂O₂ as follows:

(2) H₂O₂ → 2 H⁺ + O₂ + 2 e⁻

For samples without GL, the complex formed by the GOx enzyme and the flavin adenine dinuclotide (FAD) cofactor, GOx-FAD, oxidizes to GOx-FADH₂. In presence of GL, the GOx-FAD complex is replaced by the FAD-GL complex in the catalytic environment of the enzyme, the catalytic oxidation of the GL into gluconolactone taking place.

Figure 10b displays de variation of the peak current density for GL concentrations up to 1000 µM. The current density increases linearly with the GL concentrations until (R² = 0.966). The sensitivity of the GOx-functionalised PLA(0.8)* sensor is 0.03 µA/(cm²•µM) while the LOD is as low as 0.01 µM. These parameters are better than those reported for many enzymatic electrochemical sensors based on intrinsically conducting polymers, graphene and/or nanostructured inorganic materials. For example, the sensitivity/ LOD was 74.22 µA/(cm²•µM) / 2.9 µM for poly(3,4-ethylenedioxythiophene) (PEDOT) nanofibers, 57.3 µA/(mM•cm²) / 86.8 µM for reduced graphene oxide interdispersed with PEDOT, 0.05 µA/(cm²•µM) / 0.2 µA for carbon-decorated ZnO nanoparticles, and 0.78 µA/(cm²•µM)/5.5 µA for on Au-Ni coaxial nanorod arrays.

The effect of the potential interval was also examined for GOx-functionalised PLA(0.8)* sensors. Results for the potential interval from -0.50 to 0.50 V, which are shown in Figure 10c-d, reveals detection parameters similar to those derived from Figure 10a-b. More specifically, the sensitivity and LOD when the sensor is applied with the -0.50 to 0.50 V interval were 0.02 µA/(cm²•µM) and 0.02 µM., respectively.

### Simultaneous detection of DA and GL

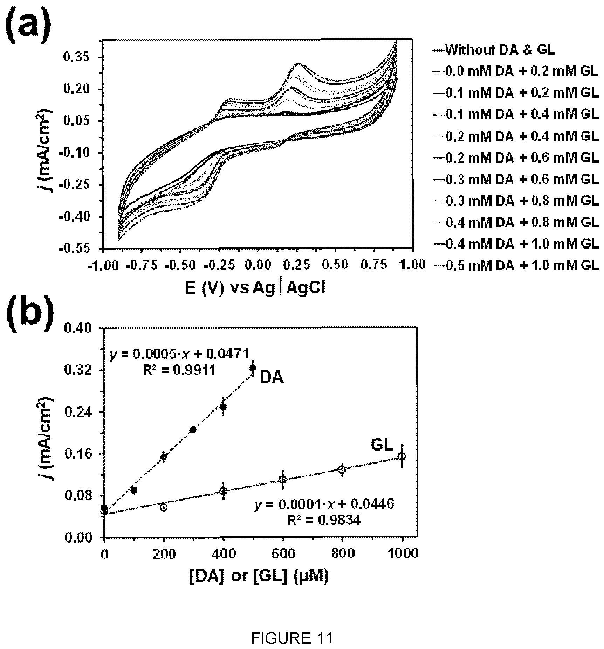

The simultaneous detection of DA and GL was performed by varying the concentration of DA and GL in the solution from 0.1 to 0.5 mM and from 0.2 to 1.0 mM, respectively. Figure 11a shows the cyclic voltammograms recorded for the potential interval from - 0.9 to 0.9 V in different DA and GL solutions. As it can be seen, multiplexed detection was achieved by measuring simultaneously the DA and GL voltammetric signals. Indeed, the utilization of two printed wells, one bare and the other functionalised with GOx, resulted in two neighbouring oxidation peaks, one for the oxidation of DA (0.3 V) and the other for the oxidation of the enzymatically produced H₂O₂ (-0.2 V), with excellent resolution and separation. This evidences that we successfully developed a manufacturing strategy for the preparation of selective PLA(0.8)* sensors for such two analytes.

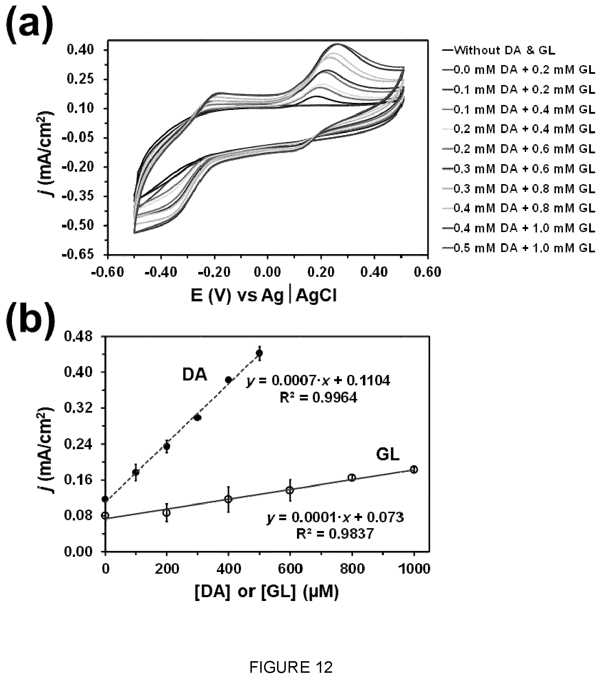

Independent linear calibration plots are displayed in Figure 11b for DA and GL (R2 = 0.991 and 0.983, respectively). For DA and GL the sensitivity is 0.5 and 0.1 µA/(cm²•µM), respectively, while the LOD is 11.8 and 71.9 µM, respectively. Although these values are higher than those obtained using solutions with a single analyte, the analytical parameters obtained for multiplexed detection are still excellent. The influence of the multidetection on the performance of the sensor does not exhibit a remarkable dependence on the interval of scanned potential. Figure 12 displays the voltammograms recorded for the potential interval from -0.5 to 0.5 V in different DA-and GL-containing solutions and the corresponding calibration plots. For DA and GL, the LOD decreases to 5.9 µM for the DA and increases to 95.5 µM, respectively.

### Example 5: Conclusions

This work describes an integrated 3-electrode electrochemical sensing device suitable for multiplexed detection, which is fabricated by 3D printing (specifically, by Fused Deposition Modelling (FDM)). Non-conductive thermoplastic PLA filaments were shaped by FDM and subsequently transformed into an electroresponsive material without the need to add any conductive materials. This was achieved by applying an electric power discharge (i.e., a plasma treatment) of 100 W for 2 min at a 0.8 mbar pressure of O₂. Spectroscopic studies and X-Ray photoelectron spectrometry (XPS) analyses evidenced that the electric discharge transformed the chemical structure of PLA, which was accompanied with drastic changes in morphology and electrochemical behaviour. Indeed, changes in the chemical structure and properties observed in PLA were significantly more important than in GCEs, which were used as simple connectors for the sensing setup. Electrochemical studies utilizing dopamine (DA)- or glucose (GL)-containing solutions proved that this plastic device can serve successfully as a versatile electrochemical sensing platform. Thus, chemically transformed PLA catalyzes the oxidation of DA and H₂O₂, the latter resulting from the enzymatic oxidation of GL. Real-time and simultaneous multiplexed detection of the two bio-analytes was achieved using prototypes with two wells, one was bare for DA sensing and the other was functionalised with GOx for GL sensing. The operational potential range, sensing parameters and manufacturing reproducibility was similar or even better than those of conventional sensors prepared using intrinsically conducting materials. The simplicity, portability, low cost, rapidity, wide commercial availability and eco-friendliness of the technology developed in this work make it a viable alternative to other manufacturing processes for the fabrication of integrated electrochemical sensors. The existing design can be readily extended to more complex geometries involving multiple wells for multiplexed sensing with a higher number of bio-analytes.

## Claims

1. A 3D-shaped electrode made of polylactic acid (PLA), wherein the PLA is modified by a plasma treatment, and wherein the 3D-shaped electrode is a 3D-printed electrode or a 3D-moulded electrode.

2. The 3D-shaped electrode according to claim 1, wherein the 3D-shaped electrode is a 3D-printed electrode, and wherein the 3D-printed electrode is 3D-printed by fused deposition modelling (FDM).

3. The 3D-shaped electrode according to any one of claims 1 or 2, wherein the plasma treatment is a plasma treatment with a low oxygen pressure plasma, wherein the low oxygen pressure is an oxygen pressure between 0.2 and 1.0 mbar, preferably wherein the oxygen pressure is 0.8 mbar.

4. The 3D-shaped electrode according to any one of claims 1 to 3, wherein the 3D-shaped electrode is in electrical contact with at least one secondary electrode for connecting to an electrochemical setup, preferably wherein the at least one secondary electrode is a glassy carbon electrode (GCEs).

5. The 3D-shaped electrode according to any one of claims 1 to 4, wherein the 3D-shaped electrode further comprises at least one functionalised region.

6. The 3D-shaped electrode according to claim 5, wherein the at least one functionalised region is a region functionalised with a Glucose Oxidase enzyme.

7. Use of a 3D-shaped electrode according to any one of claims 1 to 6 as an electrochemical sensor.

8. The use according to claim 7, wherein the electrochemical sensor is an electrochemical sensor for the detection of dopamine.

9. Use of a 3D-shaped electrode according to claim 6 as an electrochemical sensor for the multiplexed detection of glucose and dopamine.

10. A method for preparing a 3D-shaped electrode made of polylactic acid (PLA), the method comprising:
a) 3D-shaping a PLA part, wherein the 3D-shape is obtained by 3D-printing or by 3D-moulding;
b) treating the PLA part under plasma.

11. The method according to claim 10, wherein the PLA part obtained in step (a) comprises at least one hole for coupling at least one secondary electrode, preferably wherein the at least one secondary electrode is a glassy carbon electrode (GCEs), and wherein the PLA part coupled to the at least one secondary electrode is treated under plasma in step (b).

12. The method according to any one of claims 10 or 11, wherein the PLA part obtained in step (a) further comprises at least one well for enhanced detection capacity; and wherein the method comprises, after step (b), a further step (c) of coupling at least one of the wells with an enzyme or with a non-enzymatic catalytic group.

13. The method according to any one of claims 10 to 12, wherein the step (a) of 3D-shaping is a step of 3D-printing, and wherein the 3D-printing is 3D-printing by fused deposition modelling (FDM).

14. The method according to any one of claims 10 to 13, wherein the step (b) of plasma treatment is plasma treatment with a low oxygen pressure plasma, wherein the low oxygen pressure is an oxygen pressure between 0.2 and 1.0 mbar, preferably wherein the oxygen pressure is 0.8 mbar.
